# FASCICULE DE BREVET EUROPEEN

(11) **EP 3 442 659 B1**
(45) Date de publication et mention de la délivrance du brevet: **09.12.2026**
(21) Numéro de dépôt: 17722092.8
(22) Date de dépôt: 11.04.2017
(51) Int. Cl.: A61K 9/00, A61K 36/58, A61P 25/00, A61P 27/02, A61Q 7/00, A61Q 15/00, A61Q 19/00, A61Q 19/08, A61K 8/97

(54) **UTILISATION COSMÉTIQUE D'UN EXTRAIT DE KHAYA SENEGALENSIS**
KOSMETISCHE VERWENDUNG EINES EXTRAKTS AUS KHAYA SENEGALENSIS
COSMETIC USE OF A KHAYA SENEGALENSIS EXTRACT

(30) Priorité: 12.04.2016 FR 1653193
(43) Date de publication de la demande: 20.02.2019
(73) Titulaire: BASF Beauty Care Solutions France S.A.S., 69007 Lyon (FR); Centre National de la Recherche Scientifique, 75016 Paris (FR); Université Claude Bernard Lyon 1, 69622 Villeurbanne Cedex (FR)
(72) Inventeur: ANDRE, Valérie, 69420 AMPUIS (FR); BERTHELEMY, Nicolas, 54530 PAGNY SUR MOSELLE (FR); BONNET, Isabelle, 69008 LYON (FR); CADAU, Sébastien, 38080 L'ISLE D'ABEAU (FR); JEANMAIRE, Christine, 54000 NANCY (FR); MOSER, Philippe, 54130 DOMMARTEMONT (FR); RIVAL, Delphine, 69360 TERNAY (FR); ROUSSELLE, Patricia, 69005 LYON (FR)
(74) Mandataire: Cabinet Beau de Loménie
(86) Numéro de dépôt international: PCT/FR2017/050869
(87) Numéro de publication internationale: WO 2017/178751

(56) Documents cités:
- WO-A2-2012/033634
- FR-A1- 2 758 984
- ONU A. ET AL: "Effect of Aqueous Stem Bark Extract of Khaya senegalensis on Some Biochemical, Haematological, and Histopathological Parameters of Rats", JOURNAL OF TOXICOLOGY, vol. 2013, 1 January 2013 (2013-01-01), US, pages 1 - 9, XP093090717, ISSN: 1687-8191, Retrieved from the Internet <URL:https://downloads.hindawi.com/journals/jt/2013/803835.pdf> DOI: 10.1155/2013/803835
- DATABASE GNPD [online] MINTEL; 28 February 2009 (2009-02-28), "Desert Bloom Body Quencher", XP002762288, Database accession no. 1061600
- DATABASE GNPD [online] MINTEL; 31 January 2012 (2012-01-31), "Aftershave Lotion", XP002762289, Database accession no. 1693192
- DATABASE GNPD [online] MINTEL; 31 July 2013 (2013-07-31), "Scented Softness Liquid Soap for Face and Body", XP002762290, Database accession no. 2127464
- DATABASE GNPD [online] MINTEL; 30 November 2004 (2004-11-30), "MX-II Nutritive Firming Essence", XP002762291, Database accession no. 316899
- LOTTA SEPPINEN, TAINA PIHLAJANIEMI: "The multiple functions of collagen XVIII in development and disease", MATRIX BIOLOGY, 31 December 2011 (2011-12-31), pages 83 - 92, XP002762292, Retrieved from the Internet <URL:http://ac.els-cdn.com/S0945053X10001770/1-s2.0-S0945053X10001770-main.pdf?_tid=53693078-83ca-11e6-910c-00000aacb35e&acdnat=1474881748_52ceba4147484e8d3c8bf7418695213f> [retrieved on 20160926]

## Description

La présente invention concerne un nouvel ingrédient cosmétique et/ou dermatologique et son utilisation cosmétique et/ou dermatologique ainsi qu'une composition cosmétique le comprenant pour maintenir et/ou augmenter l'expression du collagène XVIII dans la peau et/ou les muqueuses et/ou le cuir chevelu et/ou les annexes cutanées, en particulier au niveau des lames basales et ainsi maintenir et/ou augmenter la fermeté de la peau et/ou des muqueuses et/ou du cuir chevelu et/ou pour diminuer la visibilité des pores cutanés.

Les lames basales, également dénommées membranes basales, ont une fonction de soutien et cohésion des différents compartiments de la peau, en particulier au niveau de l'épithélium, notamment de l'épiderme, du derme, des adipocytes et des cellules endothéliales. Une dégradation des lames basales notamment la jonction dermo-épidermique (JDE) est observée au cours du vieillissement de la peau et se traduit par une perte de la structure et des propriétés des différents compartiments. Ainsi, la dégradation de la jonction dermo-épidermique se traduit au niveau du derme par une perte de fermeté et d'élasticité, au niveau de l'épiderme par une diminution de l'épaisseur de l'épiderme et au niveau des glandes sébacées par une augmentation de la visibilité des pores cutanés due en particulier à une augmentation de leur diamètre.

Il est connu que les collagènes jouent un rôle important au niveau des lames basales notamment de la jonction dermo-épidermique. C'est en particulier le cas du collagène de type XVIII.

Le collagène XVIII est un collagène de la famille des protéoglycanes à héparane sulfate, présent dans toutes les lames basales de l'organisme et synthétisé par les kératinocytes, les adipocytes, les cellules épithéliales des glandes sudoripares, les cellules souches des follicules pileux et les cellules endothéliales. C'est en outre le seul collagène à héparane sulfate présent dans tous les compartiments de la peau.

Il joue un rôle structural important au niveau de ces lames basales, notamment la JDE, les lames basales endothéliales, épithéliales et des adipocytes. Ainsi la diminution de l'expression du collagène XVIII, notamment au cours du vieillissement de la peau comme cela est démontré dans l'exemple 2 et/ou sa dégradation, entraine une détérioration de la lame basale, une désorganisation de l'architecture de la peau et ses manifestations précédemment décrites. Ainsi, la diminution d'expression du collagène XVIII entraine un affaissement de la jonction dermo-épidermique et une diminution des échanges entre le derme et l'épiderme, une perte de fermeté et d'élasticité de la peau.

Au niveau de la JDE des glandes sébacées, la dégradation et/ou la diminution de l'expression du collagène XVIII induit un affaissement du pore cutané et une augmentation du diamètre d'ouverture du pore.

Au niveau de la lame basale de la glande sudoripare, la dégradation et/ou la diminution de l'expression du collagène XVIII induit un affaissement de la glande sudoripare ainsi qu'une augmentation de son ouverture et donc une augmentation de la transpiration. Au niveau de la lame basale du follicule pileux, la dégradation de l'expression du collagène XVIII induit une baisse des échanges nutritifs et donc une diminution de la structure et du métabolisme du bulbe pileux et une perte des poils et/ou cheveux.

En outre, la protéine de collagène XVIII est localisée au niveau des lames basales autour des adipocytes. Ainsi sa dégradation et/ou la diminution de son expression induit une déstructuration des adipocytes ce qui conduit à une perte de soutien et donc de fermeté de la peau.

Au niveau des cellules endothéliales, la dégradation et/ou la diminution de l'expression du collagène XVIII induit une diminution des apports nutritifs, induisant au niveau du derme une baisse de la synthèse des molécules structurales du derme et donc une perte de fermeté et de l'élasticité.

Le rôle structural essentiel du collagène XVIII au niveau des lames basales, en particulier la jonction dermo-épidermique, mais aussi de la lame basale des adipocytes, des cellules endothéliales, des glandes sudoripares et des follicules pileux en font une cible d'intérêt dans les domaines de la cosmétique et de la dermatologie.

De façon surprenante, les inventeurs ont découvert qu'un extrait de la plante Khaya senegalensis a la capacité d'augmenter l'expression de collagène XVIII au niveau de la peau et/ou des muqueuses et/ou du cuir chevelu et/ou des annexes cutanées, en particulier au niveau de la jonction dermo-épidermique, permettant ainsi d'augmenter à la fois la fermeté de la peau et/ou des muqueuses et/ou du cuir chevelu, mais aussi de diminuer la visibilité des pores, c'est-à-dire diminuer leur ouverture.

Khaya senegalensis est un arbre aussi appelé caïlcédrat appartenant à la famille des Méliaceae que l'on trouve dans plusieurs pays d'Afrique notamment le Bénin, le Cameroun, le Sénégal, la Guinée, le Côte-d'Ivoire ou encore le Burkina Faso. On le retrouve par ailleurs en Australie du Nord et en Nouvelle Calédonie. L'écorce de Khaya senegalensis selon l'invention provient du Burkina Faso.

Khaya senegalensis est un végétal utilisé pour la construction de pirogues à grande échelle en Afrique. En outre, ses graines sont riches en acide oléique, en faisant une huile utilisée en cuisine en Afrique de l'ouest. Khaya senegalensis est par ailleurs connue comme plante médicinale. Ainsi, les graines et les feuilles sont utilisées pour lutter contre la fièvre et les maux de tête. L'écorce en particulier est connue pour ses propriétés anti-inflammatoires, et son utilisation de fait dans le traitement de l'arthrite, lombalgies et autres douleurs articulaires, mais aussi dans le traitement de dermatoses. Des décoctions d'écorce de la plante ont été utilisées en tant que désinfectants.

La demande de brevet WO2012033634 divulgue des compositions cosmétiques anti-âges, pouvant être appliquées par voie topique, comprenant entre autre extrait végétal, un extrait de Khaya senegalensis pour inhiber la synthèse des métalloprotéinases 1 (MMP1) et stimuler la synthèse de procollagène I. Toutefois, cette demande ne divulgue pas l'utilisation cosmétique d'un extrait de Khaya senegalensis pour maintenir et/ou augmenter l'expression de collagène XVIII ni pour diminuer la visibilité des pores cutanés et/ou rendre la peau plus lisse et/ou pour maintenir et/ou augmenter la fermeté et/ou l'élasticité de la peau et/ou des muqueuses et/ou du cuir chevelu et/ou pour limiter et/ou réduire la transpiration et/ou limiter et/ou réduire la chute des cheveux et/ou des poils et/ou augmenter la croissance des cheveux et/ou des poils et/ou réduire ou limiter la production de sébum. Cet extrait n'est par ailleurs pas obtenu par extraction aqueuse.

Les demandes de brevet EP1019016 et FR2758984, divulguent en outre un complexe synergique anti-âge destiné notamment à une application topique sur la peau et comprenant un extrait déshydraté d'écorce de Khaya senegalensis, ledit complexe permettant de lutter contre le rayonnement UV, les processus inflammatoires générés par ce rayonnement et pour réduire la lipopéroxydation des fibroblastes. Cette demande ne divulgue pas l'utilisation d'un extrait de K. senegalensis pour maintenir et/ou augmenter l'expression de collagène XVIII, ni pour diminuer la visibilité des pores cutanés et/ou rendre la peau plus lisse et/ou pour maintenir et/ou augmenter la fermeté et/ou l'élasticité de la peau et/ou des muqueuses et/ou du cuir chevelu et/ou pour limiter et/ou réduire la transpiration et/ou limiter et/ou réduire la chute des cheveux et/ou des poils et/ou augmenter la croissance des cheveux et/ou des poils et/ou réduire ou limiter la production de sébum.

La demande WO2011117642 divulgue en outre des compositions de soin pour le cheveu comprenant un extrait de K. senegalensis, lesdites compositions étant adaptées pour différentes utilisations dont hydratation, action antipelliculaire ou encore effet volumisant. Cette demande de brevet ne divulgue pas d'utilisation cosmétique pour la peau et/ou les muqueuses, ni pour augmenter l'expression de collagène XVIII, ni pour augmenter la fermeté, l'élasticité de la peau et/ou des muqueuses et/ou du cuir chevelu, ni pour diminuer la visibilité des pores, ni pour limiter et/ou réduire la transpiration et/ou limiter et/ou réduire la chute des cheveux et/ou des poils et/ou augmenter la croissance des cheveux et/ou des poils et/ou réduire ou limiter la production de sébum. Ainsi, aucun document à la connaissance des demanderesses ne divulgue les utilisations de la présente invention.

Le document Mintel MX-II Nutritive firming essence décrit une composition cosmétique comprenant un extrait de *Khaya senegalensis.*

Le but de la présente invention est de fournir un tout nouvel ingrédient actif cosmétique capable, de maintenir et/ou d'augmenter l'expression de collagène XVIII au niveau de la peau et/ou des muqueuses et/ou du cuir chevelu et/ou les annexes cutanées, en particulier les glandes cutanées notamment sudoripares et sébacées et les follicules pileux. Un tel ingrédient présente l'avantage de maintenir et/ou d'augmenter la fermeté et/ou des muqueuses et/ou du cuir chevelu mais aussi de diminuer la visibilité des pores cutanés.

L'avantage de ce nouvel ingrédient actif cosmétique est d'être topiquement acceptable pour la peau et/ou les muqueuses et/ou le cuir chevelu, non toxique, disponible facilement, pouvant être fabriqué et conditionné facilement à l'échelle industrielle.

Un premier objet de la présente invention concerne donc l'utilisation cosmétique d'un extrait de Khaya senegalensis obtenu par extraction avec une solution aqueuse ne contenant pas d'alcool pour maintenir et/ou augmenter l'expression de collagène XVIII dans la peau et/ou les muqueuses et/ ou le cuir chevelu et/ou les annexes cutanées pour diminuer la visibilité des pores cutanés et/ou pour augmenter la fermeté de la peau et/ou des muqueuses et/ou du cuir chevelu.

De manière préférentielle, l'extrait selon l'invention maintient et/ou augmente l'expression du collagène XVIII dans les lames basales, préférentiellement la lame basale épithéliale préférentiellement la JDE, incluant la JDE de la peau, et/ou des muqueuses et/ou du cuir chevelu et/ou des glandes sébacées, et les lames basales des adipocytes, des cellules endothéliales, des glandes sudoripares et/ou du follicule pileux.

On entend au sens de la présente invention par « utilisation cosmétique et/ou composition cosmétique » une utilisation et/ou une composition non pharmaceutique, c'est-à-dire qui ne nécessite pas de traitement thérapeutique, c'est-à-dire destinée à toute zone de peau et/ou muqueuse et/ou du cuir chevelu dite saine.

On entend par « zone de peau saine et/ou muqueuse et/ou du cuir chevelu saine », une zone de peau ou de muqueuse ou du cuir chevelu sur laquelle est appliquée l'extrait selon l'invention et dite « non pathologique » par un dermatologue, c'est à dire ne présentant pas d'infection, de cicatrice, de maladie ou d'affection cutanée telle que candidose, impétigo, psoriasis, eczéma, acné ou dermatite, ou de plaies ou de blessures et/ou autres dermatoses.

L'extrait selon l'invention peut être appliqué sur tout ou partie de peau du corps et/ou du visage et/ou du cuir chevelu, préférentiellement les jambes, les cuisses, les bras, le ventre, le décolleté, le cou, les aisselles, les lèvres, encore préférentiellement tout ou partie du visage, préférentiellement les joues, le front, le menton, les lèvres, le contour des yeux, la zone dite « en T » du visage.

Il existe plusieurs collagène tel que le collagène de type I, III, IV, V, VI, VII, XII, XIII, XIV, XVI, XVII, XVIII, XXIV, XXIX, présents dans la peau et/ou les muqueuses et/ou le cuir chevelu. L'invention concerne le collagène XVIII. On entend par « muqueuse », la muqueuse oculaire, la muqueuse vaginale, la muqueuse uro-génitale, la muqueuse anale, la muqueuse nasale et/ou la muqueuse buccale, labiale et/ou gingivale, préférentiellement, les muqueuses oculaires et/ou buccales.

On entend par « expression de collagène XVIII », l'expression génique, c'est-à-dire l'expression des ARNm (ARN messagers) et/ou protéique du collagène XVIII. Préférentiellement, il s'agit de l'expression protéique de collagène. Préférentiellement, il s'agit de l'expression du collagène XVIII par les kératinocytes, les adipocytes, les cellules endothéliales, les cellules épithéliales des glandes sudoripares et/ou les cellules souches des follicules pileux.

De manière préférentielle, le collagène est le collagène humain, en particulier de la peau et/ou des muqueuses et/ou du cuir chevelu humain. L'expression du collagène XVIII peut être mesurée selon les méthodes classiques. L'expression protéique du collagène XVIII est préférentiellement mesurée sur des cellules en produisant c'est-à-dire les kératinocytes, les adipocytes, les cellules endothéliales, les cellules épithéliales des glandes sudoripares et/ou les cellules souches des follicules pileux.

Avantageusement, la mesure de l'expression protéique du collagène XVIII est effectuée sur kératinocytes, adipocytes, cellules endothéliales, cellules épithéliales des glandes sudoripares et/ou cellules souches des follicules pileux, en particulier sur kératinocytes, adipocytes et/ou cellules endothéliales par mesure *in vitro,* préférentiellement par mesure par microscopie confocale après immunomarquage par exemple selon la méthode telle que présentée en exemple 3.

Au sens de la présente invention, on entend par « immunomarquage », la technique consistant à fixer un anticorps fluorescent spécifique de la protéine de collagène XVIII et quantification de la fluorescence par microscopie, préférentiellement confocale.

On entend par « maintenir l'expression de collagène » empêcher une diminution du niveau d'expression génique et/ou protéique de collagène par rapport à l'expression génique et/ou protéique détectée en l'absence de l'extrait selon l'invention, notamment du niveau d'expression génique et/ou protéique de collagène que l'on observe au cours du vieillissement extrinsèque ou intrinsèque de la peau et/ou des muqueuses et/ou du cuir chevelu.

Au sens de la présente invention, on entend par « augmenter l'expression de collagène » une augmentation du niveau d'expression génique et/ou protéique de collagène, préférentiellement d'au moins 3 % en présence de l'extrait de K. senegalensis, préférentiellement d'au moins 10 %, encore préférentiellement d'au moins 20% par rapport à l'expression génique et/ou protéique détectée en l'absence de l'extrait selon l'invention. Préférentiellement, il s'agit d'une augmentation de l'expression protéique de collagène XVIII.

Dans un mode préférentiel de l'invention, il s'agit d'une augmentation de l'expression protéique de collagène XVIII mesurée dans des cellules humaines dite « normales » en produisant, c'est-à-dire non pathologiques. Avantageusement, ladite augmentation est mesurée en présence de l'extrait de K. senegalensis préparé selon l'exemple 1a).

Selon un mode avantageux de réalisation de l'invention, l'augmentation de l'expression protéique de collagène XVIII est mesurée dans des kératinocytes humains normaux, dans des adipocytes humains normaux, dans des cellules endothéliales normales, dans des cellules épithéliales des glandes sudoripares normales et/ou dans des cellules souches des follicules pileux normales, en particulier dans des kératinocytes humains normaux, dans des adipocytes humains normaux et/ou dans des cellules endothéliales normales avantageusement encore en présence de l'extrait de K. senegalensis préparé selon l'exemple 1a).

Sauf indication contraire, on entend par cellules « normales », kératinocytes « normaux », adipocytes « normaux », des cellules ne présentant pas de pathologie.

Préférentiellement, l'expression protéique de collagène XVIII est mesurée par microscopie confocale après marquage immunocytochimique à l'aide d'un anticorps anti-collagène XVIII, tel que décrit dans l'exemple 2.

Il est également divulgué l'utilisation d'un extrait de K. senegalensis selon l'invention pour maintenir et/ou augmenter l'expression génique et/ou protéique, préférentiellement protéique, de collagène XVIII, pour augmenter l'épaisseur de la jonction dermo-épidermique au niveau de la peau, des muqueuses et/ou des annexes cutanées.

On entend ici par « augmenter l'épaisseur de la jonction dermo-épidermique » augmenter sa densité et/ou augmenter les échanges entre le derme et l'épiderme et améliorer sa structure.

Un objet de la présente invention est l'utilisation cosmétique d'un extrait de K. senegalensis selon l'invention pour maintenir et/ou augmenter l'expression génique et/ou protéique, préférentiellement protéique, de collagène XVIII pour diminuer la visibilité des pores cutanés et/ou pour maintenir et/ou augmenter la fermeté de la peau et/ou des muqueuses et/ou du cuir chevelu.

On entend au sens la présente invention par « diminuer la visibilité des pores cutanés » resserrer les pores cutanés, c'est à dire diminuer le diamètre d'ouverture des pores, et/ou la densité et/ou l'aire des pores à la surface de la peau, et/ou prévenir la dilatation des pores cutanés. L'extrait selon l'invention permet donc pour diminuer l'ouverture et/ou la densité des pores à la surface de la peau.

La visibilité des pores de la peau peut être mise en évidence in vivo par une évaluation dite « scoring » par un dermatologique sur une zone prédéfinie après application d'une composition comprenant l'extrait selon l'invention. Elle peut également être mise en évidence par une méthode instrumentale objective par une analyse d'image permet d'extraire et de quantifier des paramètres spécifiques des photographies hautes résolution en configuration polarisée croisée du visage des volontaires avant et après application d'une composition comprenant l'extrait selon l'invention. La densité des pores cutanés peut également être mesurée *in vivo* par imagerie notamment par la technique de projection de franges, en mesurant le paramètre dit de curvature, dans les conditions décrites en particulier dans l'exemple 5b).

Dans un mode de réalisation préféré de l'invention, l'extrait de K. senegalensis selon l'invention est en quantité efficace pour diminuer la visibilité des pores de la peau d'au moins 10%, préférentiellement d'au moins 20%, après 28 jours d'application d'une crème comprenant l'extrait selon l'invention, avantageusement l'extrait de K. senegalensis préparé dans les conditions décrites dans l'exemple 1a), préférentiellement formulé sous forme d'ingrédient actif tel que décrit dans l'exemple 1e).

On entend au sens de la présente invention par « augmenter la fermeté » de la peau et/ou des muqueuses et/ou du cuir chevelu, une augmentation à des fins esthétiques respectivement de la fermeté de la peau et/ou des muqueuses et/ou du cuir chevelu qui ont perdu de la fermeté de façon particulière du fait d'une diminution de l'expression de collagène XVIII, encore particulièrement au niveau de la jonction dermo-épidermique.

On entend par « maintenir la fermeté» de la peau et/ou des muqueuses et/ou du cuir chevelu, empêcher le relâchement de la peau et/ou des muqueuses et/ou du cuir chevelu, de façon particulière du à une diminution de l'expression de collagène XVIII, encore particulièrement au niveau de la jonction dermo-épidermique.

La fermeté de la peau et/ou des muqueuses et/ou du cuir chevelu peut être mesurée selon les méthodes classiques connues de l'homme de l'art, notamment par mesure *in vivo à* l'aide d'un cutomètre, d'un ou encore d'un Tonoderm^{™} ou d'un DynaSKIN associé à un dermaTOP.

L'extrait de K. senegalensis selon l'invention est un extrait cosmétique et/ou dermatologique topiquement acceptable.

Au sens de la présente invention, on entend par « topiquement acceptable », un ingrédient adapté à une application par voie topique, non toxique, non irritant pour la peau et/ou les muqueuses et/ou le cuir chevelu, n'induisant pas de réponse allergique, qui n'est pas instable sur le plan chimique.

L'utilisation de l'extrait selon la présente invention peut être par voie orale ou topique. Avantageusement, elle est par voie topique. Au sens de la présente invention, on entend par « voie topique », l'application locale directe et/ou la vaporisation d'un l'ingrédient sur la surface de la peau et/ou des muqueuses et/ou du cuir chevelu.

L'extrait selon l'invention est un extrait d'écorce.

L'extrait peut être obtenu par les méthodes d'extraction de végétaux connus dans le domaine, par exemple par macération d'au moins une partie de la plante de préférence entre 1% et 30% (p/p) en poids, préférentiellement entre 10% et 20% (p/p) en poids, par rapport au poids total de la partie de plante et du solvant, dans une solution aqueuse ne contenant pas d'alccol. L'extrait est obtenu par extraction aqueuse.

L'extrait selon l'invention peut donc être obtenu par extraction d'une quantité de 10% à 20% en poids d'écorce par rapport au poids total d'écorce et de solvant, préférentiellement l'eau comme unique solvant.

Au sens de la présente invention on entend par «extrait obtenu par extraction aqueuse» tout extrait obtenu par extraction avec une solution aqueuse contenant plus de 60 % en poids, avantageusement au moins 70% en poids, en particulier au moins 80% en poids, plus particulièrement au moins 90% en poids, de façon particulière au moins 95% en poids, d'eau par rapport au poids total de la solution aqueuse, ladite solution aqueuse ne contenant pas d'alcool, plus particulièrement ne contenant que de l'eau.

L'extrait peut être obtenu par extraction à une température allant de 4°C à 95°C, préférentiellement de 20°C à 95°C, encore préférentiellement de 50 à 95°C, de préférence de 70°C à 90°C. Dans un mode de réalisation préférentiel, l'extraction est effectuée à 85°C.

L'extraction peut être conduite durant une période de 1 heure à 24 heures, préférentiellement de 1 heure à 12 heures, encore préférentiellement de 1 heure à 6 heures. Avantageusement, l'extraction est effectuée durant une période de 1 heure.

Dans un mode de réalisation avantageux de l'invention, l'extraction est réalisée en 2 phases de températures d'extraction successives, une 1^{ère} phase d'extraction à une température allant de 4°C à 95°C, préférentiellement allant de 20°C à 70°C, avantageusement à une température de 50 °C, durant une période de 1 heure à 12 heures, préférentiellement durant une période de 4 heures, suivie d'une seconde phase d'extraction à une température allant de 50°C à 95°C, préférentiellement de 85°C, durant une période de 1 heure.

L'extrait aqueux peut être décoloré par tout moyen, notamment à l'aide de charbon actif ou de terres décolorantes, auquel cas le procédé comprend généralement une étape supplémentaire de réajustement du pH de l'extrait. En variante ou en plus, le procédé décrit ci-dessus peut comprendre une étape de désodorisation de l'extrait selon toute technique connue de l'homme de l'art, avant ou après l'étape de décoloration. Selon un mode de réalisation, l'extrait éventuellement décoloré et/ou désodorisé peut ensuite être concentré par évaporation ou déshydraté par tout moyen, notamment par atomisation ou lyophilisation. Un adjuvant peut être ajouté avant ou après séchage, préférentiellement la maltodextrine, et préférentiellement avant déshydratation dans l'extrait liquide, dans des proportions allant de 20 à 80% en poids de maltodextrine par rapport au poids total de l'extrait sec obtenu après déshydratation.

Ainsi, dans un mode de réalisation particulièrement avantageux de l'invention, l'extrait est obtenu par extraction dans l'eau comme unique solvant d'une quantité de 20% en poids d'écorce de Khaya senegalensis par rapport au poids total d'écorce et d'eau, à une température de 50°C durant une période de 4 heures, puis à une température de 85°C durant une période de 1 heure. L'extrait ainsi obtenu est ensuite centrifugé et le culot éliminé. L'extrait est alors refroidi à une température de 4°C, filtré et de la maltodextrine est alors ajoutée. L'extrait est stérilisé (UHT) puis atomisé, dans les conditions décrites dans l'exemple 1a).

Dans un autre mode de réalisation, l'extrait selon l'invention est obtenu par extraction dans l'eau comme unique solvant d'une quantité de 10% en poids d'écorce de Khaya senegalensis par rapport au poids total d'écorce et d'eau, à une température de 50°C durant une période de 4 heures puis à une température de 85°C durant une période de 1 heure. L'extrait est centrifugé, le culot éliminé puis l'extrait est refroidi à une température de 4°C. L'extrait liquide obtenu est filtré et de la maltodextrine ajoutée.

L'extrait est stérilisé (UHT) puis atomisé, dans les conditions décrites dans l'exemple 1b).

Dans encore un autre mode de réalisation, l'extrait peut être obtenu dans les conditions décrites dans l'exemple 1a) puis dilué dans un mélange d'eau et de glycérine, en quantité finale de 1,5% en poids par rapport au poids total d'extrait, d'eau et de glycérine, dans les conditions décrites dans l'exemple 1e).

L'extrait de K. senegalensis peut être utilisé seul en tant qu'ingrédient actif cosmétique et/ou dermatologique, ou compris dans une composition cosmétique et/ou dermatologique. Lorsqu'il est utilisé seul sous forme d'ingrédient actif, l'extrait selon l'invention est préférentiellement soluble et dissout dans un solvant notamment polaire, tel que l'eau, un alcool, un polyol, un glycol, ou un de leurs mélanges, en présence ou non de glycérine. Préférentiellement, l'extrait est dissout dans un mélange d'eau et de glycérine pour réaliser un ingrédient cosmétique facilement formulable. Avantageusement dans ce cas, l'extrait est réalisé selon le protocole décrit dans l'exemple 1e).

La présente invention a donc également pour objet l'utilisation de l'extrait selon l'invention par voie topique sur la peau et/ou les muqueuses et/ou le cuir chevelu seul ou compris dans une composition cosmétique comprenant au moins un excipient cosmétiquement acceptable.

L'extrait de K. senegalensis selon l'invention est préférentiellement présent dans la composition à une concentration de 1.10⁻⁴% à 10% en poids, préférentiellement entre 1.10⁻⁴% et 5% en poids, encore avantageusement entre 1.10⁻³% et 3% en poids par rapport au poids total de la composition, en particulier entre 0,001 et 0,1 % en poids par rapport au poids total de la composition.

Dans un mode de réalisation, la composition cosmétique contenant l'extrait selon l'invention est utilisée pour maintenir et/ou augmenter l'expression génique et/ou protéique, préférentiellement protéique, de collagène XVIII, dans la peau et/ou les muqueuses et/ou le cuir chevelu et/ou les annexes cutanées en particulier les glandes cutanées notamment sudoripares et sébacées et les follicules pileux , en particulier dans les lames basales, préférentiellement la JDE, en particulier de la peau, et/ou des muqueuses et/ou du cuir chevelu et/ou des glandes sébacées, et/ou les lames basale des adipocytes, des cellules endothéliales, des glandes sudoripares et/ou des follicule pileux, préférentiellement pour augmenter l'épaisseur de la jonction dermo-épidermique au niveau de la peau et/ou des muqueuses et/ou du cuir chevelu, pour maintenir et/ou augmenter la fermeté de la peau et/ou des muqueuses et/ou du cuir chevelu,et/ou pour diminuer la visibilité des pores cutanés.

La composition cosmétique peut en outre renfermer un ou plusieurs excipients cosmétiquement acceptables choisis parmi des agents tensioactifs et/ou émulsifiants, des conservateurs, des agents tampons, des agents chélatants, des dénaturants, des agents opacifiants, des ajusteurs de pH, des agents réducteurs, des agents stabilisants, des épaississants, des gélifiants, des polymères filmogènes, des charges, des agents matifiants, des agents de brillance, des pigments, des colorants, des parfums, et leurs mélanges. Le CTFA (Cosmetic Ingrédient Handbook, Second Edition (1992)) décrit différents excipients cosmétiques adaptés à une utilisation dans la présente invention.

Avantageusement, le ou les excipients sont choisis dans le groupe comprenant les polyglycérols, les esters, les polymères et dérivés de cellulose, les dérivés de lanoline, les phospholipides, les lactoferrines, les lactoperoxidases, les stabilisants à base de sucrose, la vitamine E et ses dérivés, les gommes de xanthane, les cires naturelles et synthétiques, les huiles végétales, les triglycérides, les insaponifiables, les phytostérols, les silicones, les hydrolysats de protéines, les betaines, les aminoxides, les extraits de plantes, les esters de saccharose, les dioxydes de titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, la triisononanoine, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les triglycérides caprique/caprylique, le cétéaryl octanoate, le dibutyl adipate, l'huile de pépins de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, la cire d'abeille, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée, et leurs mélanges.

La composition cosmétique selon l'invention peut être choisie parmi une solution, aqueuse ou huileuse, une crème ou un gel aqueux ou un gel huileux, notamment un gel douche, un lait, une émulsion, une microémulsion ou une nanoémulsion, notamment huile-dans-eau ou eau-dans huile ou multiple ou siliconée, un masque, un sérum, une lotion, un savon liquide, un pain dermatologique, une pommade, une mousse, un patch, un produit anhydre, de préférence liquide, pâteux ou solide, par exemple sous forme de poudres de maquillage, de bâtonnet ou de stick, notamment sous forme de rouge à lèvre. De façon avantageuse, il s'agit d'une crème ou d'un sérum.

La composition utilisée selon l'invention peut en outre contenir des ingrédients actifs cosmétiques conduisant à un effet complémentaire ou synergique tels que des actifs anti-âge. On citera parmi ces actifs des actifs stimulant la synthèse des macromolécules du derme ou empêchant leur dégradation, des agents stimulant la prolifération des kératinocytes, des agents apaisants, hydratants, ou encore des agents actifs sur la régulation de la taille des pores et/ou leur ouverture.

Parmi les actifs anti-âges, on peut citer :
- un agent stimulant la synthèse de fibronectine, en particulier un extrait de maïs, un tel extrait étant notamment commercialisé par BASF Beauty Care Solutions France sous le nom Deliner^{™} et le palmitoyl pentapeptide commercialisé par la société SEDERMA sous la dénomination commerciale Matrixil^{®} ;
- un agent stimulant la formation des fibres élastiques comme un extrait d'Origanum majorana commercialisé sous le nom Dermagenist^{™} par la Demanderesse
- un agent stimulant l'expression du perlécane et du dystoglycane dans la matrice extracellulaire et/ou dans la membrane basale épithéliale comme par exemple un extrait de Polygonum bistorta commercialisé sous le nom Perlaura^{™} par BASF Beauty Care Solutions France.
- un agent de protection du facteur de croissance des fibroblastes (FGF2) de la matrice extracellulaire contre sa dégradation et/ou sa dénaturation, notamment un extrait d'Hibiscus Abelmoscus tel que décrit dans la demande de brevet au nom de la BASF Beauty Care Solutions France déposée sous le numéro FR0654316 et commercialisé par BASF Beauty Care Solutions France sous le nom Linefactor ^{™} et/ou un agent de stimulation de croissance des fibroblastes par exemple un extrait de soja fermenté contenant des peptides, connu sous le nom de Phytokine^{™} commercialisé par BASF Beauty Care Solutions France et également décrit dans la demande de brevet EP1119344B1 (Laboratoires Expanscience), et préférentiellement une combinaison de ces deux extraits ;
- un agent stimulant la synthèse de laminine, en particulier un extrait de malt modifié par biotechnologie, un tel extrait étant notamment commercialisé par BASF Beauty Care Solutions France sous le nom Basaline^{™} ;
- un agent stimulant l'expression et/ou l'activité de la Hyaluronane synthase 2 (HAS2) tels que les extraits végétaux décrits dans la demande de brevet FR2 893 252 Al et en particulier un extrait aqueux de Galanga (Alpinia galanga) et commercialisé par BASF Beauty Care Solutions France sous le nom Hyalufix^{™};
- un agent stimulant la synthèse de lysyl oxydase like (LOXL) tel qu'un extrait de Geophila cordifolia et ceux décrits dans la demande de brevet FR2855968, et en particulier un extrait d'aneth et commercialisé par BASF Beauty Care Solutions France sous le nom Lys'lastine ^{™};
- un agent stimulant la synthèse d'ATP intracellulaire, notamment un extrait d'algue Laminaria digitata ;
- un actif stimulant la synthèse des glycosaminoglycanes, tels que le produit de fermentation du lait ;
- un actif stimulateur de collagène tel que le rétinol et/ou la vitamine C ;
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, l'extrait de feuilles d'Argania spinosa commercialisé par BASF Beauty Care solutions France SAS sous le nom Arganyl^{™}; le lycopène ; les isoflavones, la quercetine, le kaempferol, l'apigénine.
- un agent de regonflement notamment les sphères de comblement d'acide hyaluronique commercialisé par BASF Beauty Care Solutions France sous le nom de Hyaluronic Filling Spheres ^{™}.
- un agent pour augmenter l'expression de LOX pour augmenter l'architecture de l'épiderme comme par exemple un extrait de Cichorium intybus commercialisé sous le nom de LOX-AGE^{™} par BASF Beauty Care Solutions France.
- un agent pour augmenter la déglycation du collagène et/ou augmenter l'expression du collagène de type I tel qu'une combinaison d'un extrait de feuilles de Salvia miltiorrhiza et de niacine commercialisé par BASF Beauty Care Solutions France sous le nom CollRepair^{™}.
- un agent stimulant la synthèse de lumican et de collagène tel qu'un tétrapeptide synthétique acetyl Gln Asp Val His commercialisé par BASF Beauty Care Solutions France sous le nom Dermican^{™}et décrit dans la demande de brevet WO2005120554A1.
- un agent de protection et de stimulation de l'élastine, du collagène comme l'extrait de feuilles de Manilkara multinervis commercialisé par BASF Beauty Care Solutions France sous le nom Elestan^{™} et l'extrait de racine de Eperua falcata commercialisé par BASF Beauty Care Solutions France sous le nom Eperuline^{™}
- un agent anti-taches pigmentaire notamment par inhibition de synthèse de mélanine tel que le complexe synergique d'extrait de Pisum sativum et de sucrose dilaurate commercialisé par BASF Beauty Care Solutions France sous le nom Actiwhite^{™}, ou l'acide hydroxyphenoxy propionique commercialisé par BASF Beauty Care Solutions France sous le nom Radianskin^{™}.

Les agents stimulant la prolifération des kératinocytes, préférentiellement utilisables dans la composition selon l'invention, comprennent notamment les rétinoïdes tels que le rétinol et ses esters, dont le palmitate de rétinyle, et le phloroglucinol. Les agents stimulant la différenciation des kératinocytes comprennent par exemple les minéraux tels que le calcium et les lignanes tels que le sécoisolaricirésinol, ainsi que l'extrait d'Achillea millefollium commercialisé sous le nom de Neurobiox^{™} par BASF Beauty Care Solutions France.

Comme agents apaisants préférentiellement utilisables dans la composition selon l'invention, on peut citer : les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de Pueraria lobata commercialisé sous le nom Inhipase^{®} par BASF Beauty Care Solutions France SAS, les extraits de Theobroma cacao.

Parmi les agents actifs sur la régulation de la taille des pores et/ou leur ouverture et/ou sur la production de sébum, on citera à titre d'exemple un extrait de Cichorium intybus commercialisé sous le nom de LOX-AGE^{™} par BASF Beauty Care Solutions France, ou de la sarcosine synthétique commercialisée sous le nom de Mat-XS^{™} Clinical et/ou un extrait d'Orthosiphon stamineus tel que décrit dans la demande de brevet WO2010063674 au nom de BASF Beauty Care Solutions France et commercialisé sous le nom MAT XS^{™} Bright.

Comme agents hydratants préférentiellement utilisables dans la composition selon l'invention, on peut citer : une combinaison de pullulan, de hyaluronate de sodium et d'alginate de sodium tel que celle commercialisée par BASF Beauty Care Solutions France sous le nom Patch2O^{™}.

Dans un mode de réalisation de la présente invention, l'utilisation est caractérisée en ce que l'extrait de K. senegalensis est appliqué sur tout ou partie du visage et/ou sur les jambes, les cuisses, les bras, le ventre, le décolleté, le cou, les aisselles, encore préférentiellement tout ou partie du visage, préférentiellement les joues, le front, le menton, le contour des yeux , la zone dite « en T » du visage.

Des exemples faisant référence à la description de l'invention sont présentés ci-après.

### Liste des figures :

Figure 1 : Evaluation des propriétés biomécaniques de la peau, expression de la déformation résiduelle de la peau et de la composante visco-élastique.
Figure 2 : Coefficients de mesure *in vivo* de l'élasticité cutanée

### Exemple 1 : préparation de différents extraits de Khava senegalensis

**Exemple 1a)** : Une quantité de 20% en poids d'écorce de la plante Khaya senegalensis par rapport au poids total d'écorce et d'eau a été broyée puis extraite dans l'eau comme unique solvant à une température de 50°C durant une période de 4 heures puis à une température de 85°C durant une période de 1 heure. L'extrait a ensuite été centrifugé et le culot est éliminé. L'extrait est alors refroidi à une température de 4°C. L'extrait liquide obtenu a été filtré et de la maltodextrine a été ajoutée (en quantité tel que la maltodextrine représente 50% en poids par rapport au poids total du mélange de extrait liquide et maltodextrine obtenu après déshydratation). L'ensemble a été stérilisé UHT puis atomisé.

**Exemple 1b)** : Une quantité de 10% en poids d'écorce de la plante Khaya senegalensis par rapport au poids total de l'écorce et de l'eau a été broyée puis extraite dans l'eau comme unique solvant à une température de 50°C durant une période de 4 heures, puis à une température de 85°C durant une période de 1 heure. L'extrait a ensuite été centrifugé puis refroidi à une température de 4°C. L'extrait liquide obtenu a été filtré, et de la maltodextrine ajoutée. L'extrait a été stérilisé UHT puis atomisé.

**Exemple comparatif 1c)** : Une quantité de 10% en poids de feuilles de la plante par rapport au poids total de feuilles et d'eau a été broyée puis extraite dans l'eau comme unique solvant à une température de 50°C durant une période de 4 heures puis à une température de 85°C durant une période de 1 heure. L'extrait a ensuite été centrifugé puis refroidi à une température de 4°C. L'extrait liquide obtenu a été filtré, séché et de la maltodextrine ajoutée. L'extrait a été stérilisé UHT puis atomisé.

**Exemple comparatif 1d)** : Une quantité de 20% en poids d'écorce de la plante Khaya senegalensis par rapport au poids total d'écorce et du mélange de solvant ci-après a été broyée puis extraite dans un mélange eau/éthanol (70,30 ; v/v) à une température de 85°C, durant une période de 1 heure. L'extrait a ensuite été centrifugé puis le culot a été éliminé. La maltodextrine a été ajoutée. L'extrait a été atomisé.

**Exemple 1e)** : Une quantité de 20% en poids d'écorce de la plante Khaya senegalensis par rapport au poids total d'écorce et d'eau a été broyée puis extraite dans l'eau comme unique solvant à une température de 50°C durant une période de 4 heures puis à une température de 85°C durant une période de 1 heure. L'extrait a ensuite été centrifugé et le culot est éliminé. L'extrait est alors refroidi à une température de 4°C. L'extrait liquide obtenu a été filtré et de la maltodextrine a été ajoutée (en quantité tel que la maltodextrine représente 50% en poids par rapport au poids total du mélange de extrait liquide et maltodextrine obtenu après déshydratation). L'ensemble a été stérilisé UHT puis atomisé. L'extrait est ensuite formulé sous forme d'ingrédient actif cosmétique comme suit : L'extrait atomisé est ainsi dilué dans un mélange d'eau et de glycérine en une quantité de 1,5% en poids par rapport au poids total du mélange eau/glycérine/extrait et 80% de glycérine en poids par rapport au poids total du mélange eau/glycérine/extrait puis filtré. L'extrait obtenu est un extrait liquide.

### Exemple 2 : Mise en évidence de la diminution de l'expression de collagène XVIII au cours du vieillissement de la peau

*Protocole:* Des échantillons de peau du visage de 6 donneurs d'âge différents (de 10 à 69 ans) dits « normaux », c'est-à-dire ne présentant pas de pathologie, ont été collectés puis congelés à -80°C. Les échantillons ont été découpés puis fixés par un mélange méthanol/acétone durant une période de 10 minutes, rincés au tampon PBS (Phosphate Buffered Saline). Les échantillons ont ensuite été incubés en présence d'un anticorps primaire anti-collagène XVIII (anticorps ciblant spécifiquement la partie collagénique du collagène XVIII et pas la partie endostatine) à température ambiante. Après rinçage au tampon PBS, les échantillons ont été incubés avec un anticorps secondaire de lapin contenant un marqueur fluorescent Cy3. Après rinçage, les noyaux cellulaires ont été marqués avec le réactif DAPI (4',6'-diamidino-2-phénylindole). L'observation et la quantification de la diminution de l'expression protéique du collagène XVIII présent au niveau de la jonction dermo-épidermique est faite à l'aide d'un microscope confocale et quantifiée par analyse d'image (3 biopsies par donneurs (n=3) et 5 champs par biopsie). (ET : Ecart-type).

### Résultats :

**Tableau 1**

| | **MOY** | **ET** |
|---|---|---|
| Donneurs âgés de 10 ans | 36,81 | 11,79 |
| Donneurs âgés de 15 ans | 27,07 | 4,29 |
| Donneurs âgés de 30 ans | 20,60 | 8,79 |
| Donneurs âgés de 44 ans | 16,51 | 3,19 |
| Donneurs âgés de 60 ans | 14,26 | 2,72 |
| Donneurs âgés de 69 ans | 13,34 | 3,98 |

*Conclusion :* les résultats ont montré une diminution de l'expression de collagène XVIII avec l'âge des donneurs, démontrant le lien entre l'expression de collagène XVIII et le vieillissement de la peau.

### Exemple 3 : Augmentation de l'expression de collagène XVIII en présence d'un extrait de Khaya senegalensis

### Exemple 3a) Augmentation de l'expression de collagène XVIII dans des kératinocytes

*Protocole :* Des kératinocytes humains dits « normaux », c'est-à-dire ne présentant pas de pathologie issus du sein d'une donneuse saine de 36 ans (femme, sein) ont été cultivés dans un milieu défini (KSFM) durant une période de 48 heures en présence de 2 concentrations finales différentes de l'extrait de Khaya senegalensis préparé selon l'exemple 1a) puis le milieu cellulaire éliminé. Le tapis cellulaire obtenu a été fixé (tampon Phosphate Buffered Saline (PBS)/paraformaldéhyde) puis perméabilisé (tampon PBS/Triton).

Le dosage du collagène XVIII a été effectué avec un anticorps anti-collagène XVIII, dilué au 1/500 dans une solution de tampon PBS contenant de la Sérum Albumine Bovine (SAB). Après une période de 90 minutes, un anticorps secondaire couplé à la fluorescéine dilué au 1/200 est appliqué durant une période de 2 heures à l'obscurité. Après séchage, le mélange a été resolubilisé avec une solution d'hydroxyde d'ammonium. La fluorescence a été mesurée (ENVision, PerkinElmer). Les résultats de fluorescence ont été normalisés par rapport à la fluorescence obtenue avec le même milieu cellulaire en l'absence de l'extrait de Khaya senegalensis (Contrôle) et ont été rapportés à la viabilité cellulaire obtenue dans chaque condition. Les résultats présentés correspondent à la moyenne de 6 essais (n=6). (ET : Ecart-type).

### Résultats :

**Tableau 2**

| | MOY | ET |
|---|---|---|
| Contrôle | 100 | 14,0 |
| Extrait de K. senegalensis 3 x10⁻³ % (p/v milieu) Ex. 1a) | 154,0 | 18,6 |
| Extrait de K. senegalensis 1,5 x10⁻³ % (p/v milieu) Ex. 1a) | 149,9 | 11,1 |

*Conclusion :* l'extrait de Khaya senegalensis selon l'invention a augmenté significativement l'expression protéique de collagène XVIII par des kératinocytes humains d'au moins +49% versus contrôle non traité (Test d'analyse statistique Donnett p<0,001).

### Exemple 3b) Augmentation de l'expression de collagène XVIII dans des adipocytes

*Protocole :* Des pré-adipocytes humains subcutanés dits normaux, c'est-à-dire ne présentant pas de pathologie issus d'un donneur féminin ont été cultivés dans un milieu de croissance spécifique, durant une période de 3 jours à une température de 37 °C sous 5 % atmosphère CO₂. Lorsque le stade de saturation du tapis cellulaire a été atteint, le milieu a été remplacé par un milieu de différenciation, dans lequel a été ajouté l'extrait de Khaya senegalensis selon l'invention préparé dans les conditions décrites dans l'exemple 1a), à différentes concentrations finales dans le milieu (p/v), et cultivé durant une période de 6 jours.

L'expression protéique du collagène XVIII a été évaluée par immunocytochimie. Les cellules cultivées ci-dessus-ont été fixées avec de l'acétone à -20°C durant une période de 10 minutes puis rincées (tampon PBS) et placées dans un sérum durant une période de 30 minutes à 37°C et rincées (tampon PBS). Un anticorps primaire anti-collagène XVIII dilué 1/200 a ensuite été incubé durant une période de 2 heures à température ambiante. Après rinçage, un anticorps secondaire dilué 1/200 a été incubé durant une période de 45 minutes à température ambiante et à l'abri de la lumière. Le milieu a ensuite été éliminé puis les cellules ont été contre-colorées (Bleu Evans) durant une période de 10 minutes puis rincé (PBS). Les observations ont été effectuées par microscopie confocale. La quantification du collagène XVIII a été réalisée après analyse d'image. L'augmentation de l'expression protéique du collagène XVIII est proportionnelle au pourcentage d'occupation dudit collagène sur les images. Les résultats du tableau 3 sont exprimés par la moyenne du pourcentage d'occupation du collagène XVIII (n=6). (ET : Ecart-type)

### Résultats :

**Tableau 3**

| | MOY | ET |
|---|---|---|
| Contrôle | 4,1 | 0,4 |
| Extrait de K. senegalensis 1 x10⁻⁴ % (p/v milieu) Ex. 1a) | 12,9 | 0,8 |
| Extrait de K. senegalensis 3 x10⁻⁵ % (p/v milieu) Ex. 1a) | 8,9 | 0,8 |

*Conclusion :* l'extrait de Khaya senegalensis selon l'invention a augmenté de manière significative l'expression protéique du collagène XVIII par les adipocytes humains d'au moins +117,1% et jusqu'à +214,6% versus contrôle non traité (analyse statistique Shapiro-Wilk p<0.001)

### Exemple 3c) Augmentation de l'expression de collagène XVIII dans des cellules endothéliales

### Protocole :

Des cellules endothéliales humaines dites normales, c'est-à-dire ne présentant pas de pathologie issus d'un donneur de 62 ans sain ont été cultivées dans un milieu de croissance spécifique (EMB-2) durant une période de 5 jours à une température de 37 °C sous 5 % atmosphère CO_{2.} Les cellules sont ensuite ensemencées sur Labtecks (millicell EZ slid) 8 puits, toujours dans un milieu de croissance spécifique dans lequel a été ajouté l'extrait de Khaya senegalensis selon l'invention préparé dans les conditions décrites dans l'exemple 1a), à différentes concentrations finales dans le milieu (p/v), et cultivé durant une période de 3 et 7 jours.

L'expression protéique du collagène XVIII a été évaluée par immunocytochimie. Les cellules cultivées ci-dessous ont été fixées avec de l'acétone à -20°C durant une période de 10 minutes puis rincées (tampon PBS) et placées dans un sérum durant une période de 30 minutes à 37°C et rincées (tampon PBS). Un anticorps anti-collagène XVIII dilué 1/500 a ensuite été incubé durant une période de 2 heures à température ambiante. Un anticorps secondaire (Anti-rabbit Alexas 488, Invitrogen) dilué 1/250 a été incubé durant une période de 45 minutes à température ambiante et à l'abri de la lumière. Le milieu a ensuite été rincé puis coloré (Bleu Evans) durant une période de 10 minutes puis rincé (PBS). L'observation a été effectuée par microscopie confocale. La quantification du collagène XVIII a été obtenue après analyse d'image. L'augmentation de l'expression protéique du collagène XVIII est proportionnelle au pourcentage d'occupation dudit collagène sur les images.

### Résultats :

Les résultats obtenus ont montré une augmentation significative de l'expression du collagène XVIII par les cellules endothéliales.

### Exemple 4: Mise en évidence in vitro de l'amélioration des propriétés biomécaniques de la peau en présence d'un extrait de Khaya senegalensis.

### Exemple 4a) Amélioration de la déformation résiduelle de modèles de peaux reconstruites

*Protocole :* Lors de chaque étape de renouvellement du milieu, l'extrait de Khaya senegalensis selon l'invention préparé dans les conditions décrites dans l'exemple 1a) a été ajouté à la concentration de 0,00075% (p/v). Des kératinocytes humains dits « normaux », c'est-à-dire ne présentant pas de pathologie obtenus de biopsie de sein d'une donneuse saine âgée de 30 ans et des fibroblastes humains dits « normaux », c'est-à-dire ne présentant pas de pathologie obtenus de biopsie de sein d'une donneuse saine âgée de 18 ans ont été obtenus. Les fibroblastes ont été cultivés dans un milieu spécifique DMEM (Dulbecco's modified Eagle's medium) contenant 10% de sérum calf et des antibiotiques (Normocine, 100 µg/mL). Les kératinocytes ont été cultivés dans un milieu K-FSM (Life Technologies) contenant des antibiotiques jusqu'à sub-confluence. Des fibroblastes dermiques ont été ensemencés sur un substrat constitué de collagène, chitosan et glycosaminoglycans et ces équivalents de derme ont été cultivés durant une période de 28 jours à une température de 37°C sous atmosphère CO₂ 5%. Le milieu contenant les fibroblastes cultivés sur milieu spécifique DMEM a été supplémenté avec 10% de sérum de veau fœtal, de l'acide ascorbique (50 µg/mL) et des antibiotiques.

Les kératinocytes ont ensuite été ensemencés sur les équivalents de derme et ont été cultivés durant une période supplémentaire de 21 jours dans un milieu simple DMEM contenant de l'albumine de sérum bovin (BSA) (0,8%), de l'insuline, de l'hydrocortisone, de l'acide ascorbique et des antibiotiques. Afin de déclencher le processus de différenciation, les peaux reconstruites ont été placées à une interface air-liquide 7 jours après l'ensemencement des kératinocytes

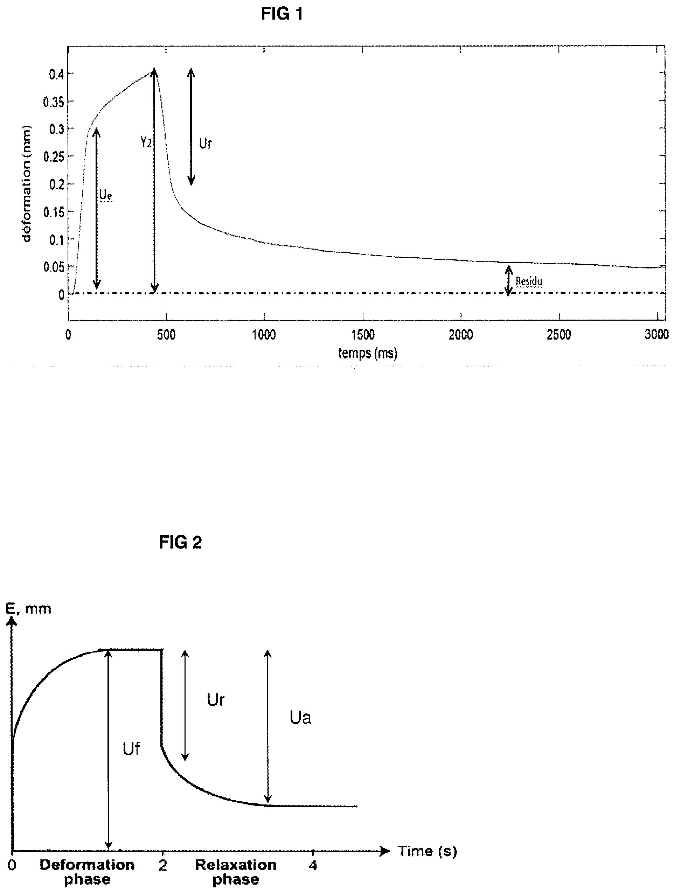

Les propriétés biomécaniques des modèles de peau reconstruites ont été mesurées par l'intermédiaire d'une méthode basée sur l'évaluation de la déformation de la peau sous un flux d'air contrôlé La déformation de la peau est suivie grâce à un laser couplé à l'équipement. Une courbe de déformation est alors obtenue (cf. figure 1). La valeur « résidu » traduit la déformation résiduelle de la peau en mm (Résidu, Figure 1). Plus ce coefficient « résidu » est faible, plus la peau est élastique et reprend sa forme initiale. Les résultats sont exprimés en mm (n=13) (ET : Ecart-type).

### Résultats :

**Tableau 4**

| | résidu (mm) | ET |
|---|---|---|
| Contrôle | 0,0451 | 0,0131 |
| Extrait de Khaya senegalensis 7,5 x10⁻⁴ % Ex. 1a) | 0,0318 | 0,0189 |

*Conclusion* : les résultats ont montré que des peaux reconstruites cultivées en présence de l'extrait de Khaya senegalensis selon l'invention ont un taux de déformation résiduelle significativement plus faible que lorsqu'elles sont cultivées en l'absence de l'extrait (test d'analyse statistique de Mann-Whitney p<0,05). L'extrait selon l'invention permet donc bien d'augmenter l'élasticité de la peau.

### Exemple 4b) Amélioration de la composante viscoélastique des peaux reconstruites en présence de l'extrait de Khaya senegalensis.

*Protocole :* les peaux reconstruites ont été cultivées dans les conditions décrites ci-dessus. L'évaluation de la composante visco-élastique des peaux est traduite par l'intermédiaire du paramètre (Y2-Ue)/Y2 (Figure 1).

Lorsque ledit paramètre diminue, l'élasticité de la peau augmente, impliquant que la peau revient plus facilement à son état initial. Les résultats sont présentés dans le tableau 5 et sont exprimés par la moyenne de 13 essais (n=13).

### Résultats :

**Tableau 5**

| | MOY | ET |
|---|---|---|
| Contrôle | 0,333 | 0,031 |
| Extrait de Khaya senegalensis 7,5 x10⁻⁴ % Ex. 1a) | 0,256 | 0,062 |

*Conclusion :* les résultats ont montré une diminution significative (test d'analyse statistique de Mann-Whitney p<0,001) du paramètre (Y2-Ue)/Y2 d'au moins de 23,1% par rapport au contrôle dans les peaux reconstruites cultivées en présence de l'extrait de Khaya senegalensis selon l'invention, traduisant une augmentation de l'élasticité des peaux reconstruites.

### Exemple 5 : Mise en évidence in vivo des effets sur la peau d'un extrait de Khava senegalensis

*Protocole :* La crème décrite dans l'exemple 6 comprenant l'extrait de Khaya senegalensis préparé selon l'exemple 1e) à une concentration finale en poids de 1% par rapport au poids total de la crème et la même crème placebo sans extrait ont été appliquées tous les jours chacune sur une moitié de visage de 25 volontaires femmes caucasiennes âgées de 53 à 65 ans et possédant une peau avec pores visibles et ayant une perte d'élasticité, durant une période de 2 mois.

### Exemple 5a) Mesure in vivo de l'élasticité de la peau

L'élasticité de la peau a été évaluée par cutométrie en prenant en compte les coefficients R2 et R7. Le coefficient R2 correspond au rapport Ua/Uf (partie comprise entre l'amplitude maximale et la capacité de déformation de la peau) (cf. figure 2), traduisant l'élasticité globale. Le coefficient R7 correspond au rapport Ur/Uf traduisant l'élasticité immédiate de la peau (Figure 2). Les résultats sont exprimés en % d'augmentation d'élasticité aux temps T28 et T56 jours, par rapport au contrôle (Crème sans extrait de K. senegalensis).

### Résultats :

**Tableau 6 : Coefficient R2 (Ua/Uf)**

| | % d'amélioration vs. Placebo Coefficient Ua/Uf (R2) |
|---|---|
| | T56 |
| Extrait de K. senegalensis 1% (p/p) | +10,70% |

*Conclusion :* Les résultats ont montré une augmentation significative (p<0.05 test t Student) de l'élasticité globale de la peau des hémivisages traités avec la crème comprenant l'extrait de Khaya senegalensis au bout de 56 jours d'application quotidienne en comparaison de la peau des hémivisages traités avec la crème placebo.

**Tableau 7 : Coefficient R7 (Ur/Uf)**

| | % d'amélioration vs placebo Coefficient Ur/Uf (R7) | |
|---|---|---|
| | T28 | T56 |
| Extrait de K. senegalensis 1% (p/p) | +9,60 | +11,60 |

*Conclusion :* les résultats ont montré une augmentation significative (p<0,05 test t de Student)de 9,6% de l'élasticité immédiate de la peau des hémivisages traités avec la crème comprenant l'extrait de Khaya senegalensis au bout de 28 jours d'application quotidienne en comparaison de la peau des hémivisages traités avec la crème placebo et de 11,60% de l'élasticité immédiate de la peau des hémivisages traités avec la crème comprenant l'extrait de Khaya senegalensis au bout de 56 jours d'application quotidienne en comparaison de la peau des hémivisages traités avec la crème placebo.

### Exemple 5b) Mesure in vivo de la densité des pores cutanés

La densité des pores a été évaluée par la méthode de projection de franges en analysant le microrelief cutané, par l'intermédiaire du paramètre dit de « curvature ». Plus ce paramètre est important, plus l'hétérogénéité de surface (pores et micro-relief cutané)est importante. Comme cette mesure a été effectuée sur les joues, on peut la corréler directement à une mesure de la densité des pores. Une diminution dudit paramètre traduit donc une diminution de la densité des pores cutanés et du microrelief cutané

*Résultats :* les résultats sont exprimés en % de réduction du paramètre curvature par rapport au placebo

**Tableau 8**

| | T28 | T56 |
|---|---|---|
| Extrait de K. senegalensis 1% (p/p) | -22,8% | -38,2% |

*Conclusion :* les résultats ont montré une diminution du paramètre d'intérêt, traduisant une diminution significative (p<0,05 test t de Student) de la densité des pores de 22,80%, en présence de la crème comprenant l'extrait de K. senegalensis selon l'invention au bout de 28 jours d'application.

### Exemple 5 c) Mesure in vivo des rides de la peau

L'efficacité de l'extrait selon l'invention sur rides de la patte d'oie a été mesurée au temps T28 (28 jours) en mesurant le volume desdites rides par la méthode de projection de franges dans les conditions déjà décrites ci-dessus (Protocole exemple 5b). Les résultats sont exprimés en % de réduction du volume des rides vs contrôle (crème sans extrait), en présence de la crème comprenant l'extrait de K. senegalensis selon l'invention

### Résultats :

**Tableau 9**

| | T56 |
|---|---|
| Extrait de K. senegalensis 1% (p/p) | -16,2 |

*Conclusion :* L'extrait de K. senegalensis a diminué le volume des rides détecté au temps T56 jours de 16,2% en comparaison du contrôle (p<0,05 test t de Student).

### Exemple 6: Exemple de composition cosmétique comprenant un extrait de Khava senegalensis

On procède selon les méthodes connues de l'homme du métier pour mélanger ensemble les différentes phases A, B, C, D ci-dessous pour préparer une composition selon la présente invention. Les proportions sont exprimées en %.

### Phase A

| | |
|---|---|
| Glycérile stéarate, PEG-100 Stéarate | 4,00 |
| Pentaerythrityl distéarate | 1,50 |
| Cétéaryl Isononanoate | 3,00 |
| Propylheptyl caprylate | 5,00 |
| Coco caprylate | 2,00 |
| Dicaprylyl carbonate | 3,00 |
| Diméthicone | 1,00 |

### Phase B

| | |
|---|---|
| Eau | 64,43 |
| Propylène glycol, phenoxyéthanol, chlorphenesine, methylparabène | 1,00 |
| Glycérine | 1,57 |
| Gomme de xanthane | 0,20 |
| Butylène glycol | 2,00 |
| Hydroxyde de sodium, eau | 0,15 |

### Phase C

| | |
|---|---|
| Carbomère | 0,15 |
| Eau | 10 |

### Phase D

| | |
|---|---|
| Extrait de Khaya senegalensis obtenu selon exemple 1 e) | 1,00 |

## Revendications

1. Utilisation cosmétique d'un extrait de Khaya senegalensis obtenu par extraction aqueuse pour maintenir et/ou augmenter l'expression de collagène XVIII dans la peau et/ou les muqueuses et/ou le cuir chevelu et/ou les annexes cutanées pour diminuer la visibilité des pores cutanés et/ou pour augmenter la fermeté de la peau et/ou des muqueuses et/ou du cuir chevelu **caractérisée en ce que** l'extrait de Khaya senegalensis est un extrait d'écorce et est obtenu par extraction avec une solution aqueuse ne contenant pas d'alcool.

2. Utilisation selon la revendication 1 pour maintenir et/ou augmenter l'expression de collagène XVIII dans la jonction dermoépidermique de la peau, et/ou des muqueuses et/ou du cuir chevelu et/ou des glandes sébacées, et/ou les lames basales des adipocytes, des cellules endothéliales, des glandes sudoripares et/ou des follicules pileux.

3. Utilisation selon l'une des revendications précédentes dans laquelle l'expression de collagène XVIII est l'expression protéique.

4. Utilisation selon l'une des revendications précédentes dans laquelle le collagène XVIII est exprimé par les kératinocytes les adipocytes, les cellules endothéliales, les cellules épithéliales des glandes sudoripares et/ou les cellules souches des follicules pileux.

5. Utilisation selon l'une des revendications précédentes dans laquelle l'extrait est utilisé par voie topique.

6. Utilisation d'un extrait selon l'une quelconque des revendications 1 à 5 telle que l'extrait de Khaya senegalensis est présent dans une composition cosmétique comprenant au moins un excipient cosmétiquement acceptable, à une concentration de 1.10⁻⁴% à 10% en poids, préférentiellement de 1.10⁻⁴% à 5% en poids, encore avantageusement de 1.10⁻³% à 3% en poids, encore préférentiellement de 0,001% et 0,1% en poids par rapport au poids total de la composition.

7. Utilisation selon l'une quelconque des revendications 1 à 6, **caractérisé en ce que** l'extrait est appliqué par voie topique sur tout ou partie du visage et/ou sur les jambes, les cuisses, les bras, le ventre, le décolleté, le cou, les aisselles..

8. Utilisation selon l'une quelconque des revendications 1 à 6, **caractérisé en ce que** l'extrait est appliqué par voie topique sur tout ou partie du visage, et préférentiellement les joues, le front, le menton, le contour des yeux, de façon particulièrement avantageuse la zone dite « en T » du visage.

## Patentansprüche

1. Kosmetische Verwendung eines durch wässrige Extraktion erhaltenen Extrakts von Khaya senegalensis zur Aufrechterhaltung und/oder Erhöhung der Expression von Kollagen XVIII in der Haut und/oder den Schleimhäuten und/oder der Kopfhaut und/oder den Hautanhangsgebilden zur Verringerung der Sichtbarkeit der Hautporen und/oder zur Erhöhung der Straffheit der Haut und/oder der Schleimhäute und/oder der Kopfhaut, **dadurch gekennzeichnet, dass** der Extrakt von Khaya senegalensis ein Rindenextrakt ist und durch Extraktion mit einer wässrigen Lösung ohne Alkohol erhalten wird.

2. Verwendung nach Anspruch 1 zur Aufrechterhaltung und/oder Erhöhung der Expression von Kollagen XVIII in der dermoepidermalen Junktionszone der Haut und/oder der Schleimhäute und/oder der Kopfhaut und/oder der Talgdrüsen und/oder Basallamina von Adipozyten, Endothelzellen, Schweißdrüsen und/oder Haarfollikeln.

3. Verwendung nach einem der vorhergehenden Ansprüche, wobei die Expression von Kollagen XVIII eine Proteinexpression ist.

4. Verwendung nach einem der vorhergehenden Ansprüche, wobei das Kollagen XVIII durch Keratinozyten, Adipozyten, Endothelzellen, Epithelzellen von Schweißdrüsen und/oder Stammzellen von Haarfollikeln exprimiert wird.

5. Verwendung nach einem der vorhergehenden Ansprüche, wobei der Extrakt topisch genutzt wird.

6. Verwendung eines Extrakts nach einem der Ansprüche 1 bis 5, so dass der Extrakt von Khaya senegalensis in einer kosmetischen Zusammensetzung, die mindestens einen kosmetisch akzeptablen Hilfsstoff umfasst, in einer Konzentration von 1,10⁻⁴ bis 10 Gew.-%, vorzugsweise von 1,10⁻⁴ bis 5 Gew.-%, bevorzugter von 1,10⁻³ bis 3 Gew.-%, noch bevorzugter von 0,001 Gew.-% und 0,1 Gew.-% vorliegt, bezogen auf das Gesamtgewicht der Zusammensetzung.

7. Verwendung nach einem der Ansprüche 1 bis 6, **dadurch gekennzeichnet, dass** der Extrakt topisch auf das gesamte oder einen Teil des Gesichts und/oder auf die Beine, Oberschenkel, Arme, den Bauch, das Dekolleté, den Hals, die Achseln aufgetragen wird.

8. Verwendung nach einem der Ansprüche 1 bis 6, **dadurch gekennzeichnet, dass** der Extrakt topisch auf das gesamte oder einen Teil des Gesichts, vorzugsweise auf die Wangen, die Stirn, das Kinn, die Augenpartie, besonders vorteilhaft auf die so genannte "T-förmige" Zone des Gesichts aufgetragen wird.

## Claims

1. The cosmetic use of a Khaya senegalensis extract obtained by aqueous extraction for maintaining and/or increasing the expression of collagen XVIII in the skin and/or mucous membranes and/or the scalp and/or the skin appendages to reduce the visibility of the skin pores and/or to increase the firmness of the skin and/or skin mucous membranes and/or scalp, wherein the Khaya senegalensis extract is an extract of the bark and is obtained by extraction with an aqueous solution that does not contain alcohol.

2. The use as claimed in claim 1 for maintaining and/or increasing the expression of collagen XVIII in the dermoepidermal junction of the skin and/or the mucous membranes and/or the scalp and/or the sebaceous glands, and/or the basal laminae of adipocytes, endothelial cells, sudoriparous glands and/or hair follicles.

3. The use as claimed in one of the preceding claims, wherein the expression of collagen XVIII is protein expression.

4. The use as claimed in one of the preceding claims, wherein the collagen XVIII is expressed by keratinocytes, adipocytes, endothelial cells, epithelial cells of the sudoriparous glands and/or stem cells of hair follicles.

5. The use as claimed in one of the preceding claims, wherein the extract is used topically.

6. The use of an extract as claimed in any one of claims 1 to 5, such that the Khaya senegalensis extract is present in a cosmetic composition comprising at least one cosmetically acceptable excipient at a concentration of from 1 x 10⁻⁴% to 10% by weight, preferably from 1 x 10⁻⁴% to 5% by weight, even more advantageously from 1 x 10⁻³% to 3% by weight, even more preferably from 0.001% to 0.1% by weight, relative to the total weight of the composition.

7. The use as claimed in any one of claims 1 to 6, wherein the extract is applied topically to all or part of the face and/or to the legs, thighs, arms, stomach, upper chest, neck or armpits.

8. The use as claimed in any one of claims 1 to 6, wherein the extract is applied topically to all or part of the face, and preferably to the cheeks, forehead, chin and eye area, particularly advantageously to the "T-zone" of the face.
